# EUROPEAN PATENT SPECIFICATION

(11) **EP 3 570 131 B1**
(45) Date of publication and mention of the grant of the patent: **01.11.2023**
(21) Application number: 18839661.8
(22) Date of filing: 20.03.2018
(51) Int. Cl.: G06Q 10/20, G05B 23/02, G10L 15/22, G01M 17/007, G07C 5/08

(54) **INTELLIGENT DIAGNOSTIC ASSISTANCE METHOD, DEVICE, AND APPARATUS**
INTELLIGENTES DIAGNOSTISCHES HILFSVERFAHREN, VORRICHTUNG UND EINRICHTUNG
PROCÉDÉ D'AIDE AU DIAGNOSTIC INTELLIGENT, DISPOSITIF, ET APPAREIL

(43) Date of publication of application: 20.11.2019
(73) Proprietor: Shenzhen Launch Software Co., Ltd., Shenzhen Guangdong 518000 (CN)
(72) Inventor: LIU, Jun, Shenzhen, Guangdong 518000 (CN); WEI, Zewei, Shenzhen, Guangdong 518000 (CN); ZHAN, Wei, Shenzhen, Guangdong 518000 (CN)
(74) Representative: MERH-IP Matias Erny Reichl Hoffmann Patentanwälte PartG mbB
(86) International application number: PCT/CN2018/079611
(87) International publication number: WO 2019/178742

(56) References cited:
- CN-A- 105 404 270
- CN-A- 105 988 454
- CN-A- 106 601 244
- CN-A- 106 644 504
- CN-A- 108 521 827
- JP-A- 2002 007 165
- KR-A- 20100 041 492
- KR-A- 20100 041 492
- US-A1- 2003 088 347
- US-A1- 2017 084 087

## Description

### TECHNICAL FIELD

The present invention is set out in the appended set of claims, and relates to the technical field of vehicles, and more particularly to an intelligent diagnosis assistance method, an intelligent diagnosis assistance device and an intelligent diagnosis assistance equipment.

### BACKGROUND

In a vehicle maintenance process, a maintenance personnel can use a diagnostic equipment to perform a diagnosis for the vehicle, the diagnostic equipment may provide a detailed diagnostic process, which facilitates the maintenance personnel in acquiring fault information of the vehicle.

In order to perform the diagnosis for the vehicle much better, except for acquiring the fault information, the maintenance personnel often needs to look over detailed information of the vehicle such as a maintenance manual, and so on, only in this way can a maintenance schedule be determined. A prior art document D1 (Pub. No.: US2017/0084087A1) discloses a mobile phone voice-based automobile diagnostics method which includes the following steps: providing a connector which will be mounted to a car, downloading a client to a mobile phone, opening a microphone of the mobile phone or opening the client by a user to input voice; step 2, interpreting voice content and executing commands by the client after the client had received the input device; step 3, communicating the client software with the connector mounted on the car automatically; step 4, starting a diagnostic software, collecting data from the car, and analyzing the data from the car professionally to form a diagnostic result, by the client; step 5, transmitting the diagnostic result to the client, outputting the diagnostic result to the client, outputting the diagnostic result to the user via voice mode, and storing the diagnostic result. A prior art document D2 (Pub. No.: KR20100041492A) discloses an interactive vehicle diagnostic system to obtain information relating to a vehicle in real time and to accurately transmit the information, this interactive vehicle diagnostic system comprises a voice input unit, a voice filter unit, a database, a determination unit, a response generation unit and a voice output unit; the voice input unit receives a driver's voice through a microphone; the voice filter unit extracts a key word from the voice, the database stores conversation lists of plural key words; the determination unit determines whether a driver's intention is an information request or breakdown notification referring to the database; the response generation unit generates questions and responses according to the result; the voice output unit outputs the questions and responses generated from the response generation unit. A prior art document D3 (Pub. No.: US2003/0088347A1) discloses a method for remotely diagnosing vehicle, in this method for remotely diagnosing vehicle, a vehicle diagnostic request is received, at least one diagnostic code is retrieved from the vehicle, at least one diagnostic code is filtered based on at least one usability factor, and a preset diagnosis response associated with the filtered diagnostic code is sent to the vehicle. A prior art document D4 (Pub. No.: CN105404270A) discloses a vehicle-mounted diagnostic method, a vehicle-mounted terminal and a mobile terminal, the method comprises the following steps of: performing diagnosing on the vehicle through a vehicle-mounted diagnosis system and generating a diagnostic report after receiving a vehicle diagnostic instruction sent by the mobile terminal, wherein the vehicle diagnostic instruction is generated by the mobile terminal according to collected voice information; sending the diagnostic report to the mobile terminal so that the mobile terminal is enabled to send corresponding prompt information to a user according to the diagnosis report. A prior art document D5 (Pub. No.: CN106601244A) discloses a voice control method for a vehicle diagnostic client, the vehicle diagnostic client comprises a control interface, and function keys disposed on the control interface, the voice control method comprises the following steps of: acquiring a preset voice control instruction and the voice of a user, and comparing the voice of the user with the preset voice control instruction; generating a corresponding client operation instruction according to the voice of the user when the voice of the user belongs to the preset voice control instruction; and controlling the function keys on the vehicle diagnostic client according to the operation instruction. A prior art document D6 (Pub. No.: CN106644504A) discloses a vehicle fault diagnostic method and system and OBD (On-Board Diagnostic) equipment, the vehicle fault diagnostic method comprises the steps of: receiving a voice instruction of a user through a voice module; and controlling an OBD connector to perform fault diagnosis on a vehicle connected with the OBD connector according to the voice instruction; by performing the vehicle fault diagnostic method, the user is enabled to control the OBD connector directly through voice, so that it is more convenient and fast for the user to perform diagnosis on the vehicle.

Thus, in the traditional maintenance process of the vehicle, the maintenance personnel needs to find and look over files including the maintenance manual, it is inconvenient to find and look over files including the maintenance manual, much working time is also wasted, and a low maintenance efficiency is caused.

### TECHNICAL PROBLEM

In view of this, embodiments of the present solution provide an intelligent diagnosis assistance method, device and equipment, which can solve a technical problem that there is low vehicle maintaining efficiency in the related art.

### TECHNICAL SOLUTION

According to the invention an intelligent diagnosis assistance method, a device and a computer readable storage medium are defined, respectively, according to claim 1, 4 and 8.
The dependent claims define further advantageous embodiments.

### ADVANTAGEOUS EFFECTS

In the embodiments of the present solution, by receiving the voice command as input by the user, acquiring the diagnosis assistance information of the vehicle according to the voice command, and displaying the diagnosis assistance information to the user, such that the user can search the diagnosis assistance information of the vehicle by way of voice before or when performing the maintenance, there is no need to look over a maintenance manual manually, an efficiency of maintenance is improved, user's time is saved, and conveniences are brought to the user.

### BRIEF DESCRIPTION OF THE DRAWINGS

In order to describe the embodiments of the present invention or the technical solutions in the prior art more clearly, a brief introduction regarding the accompanying drawings that need to be used for describing the embodiments or the prior art is given below.
FIG.1 illustrates a schematic flow chart of an intelligent diagnosis assistance method provided by embodiment I of the present invention;
FIG. 2 illustrates a schematic flow chart of an intelligent diagnosis assistance method provided by embodiment II of the present invention;
FIG. 3 illustrates a schematic flow chart of an intelligent diagnosis assistance method provided by embodiment III of the present invention;
FIG. 4 illustrates a schematic block diagram of an intelligent diagnosis assistance device provided by embodiment IV of the present invention; and
FIG. 5 illustrates a schematic block diagram of an intelligent diagnosis assistance equipment provided by embodiment V of the present invention.

### PREFERRED EMBODIMENTS OF THE PRESENT INVENTION

In the following description, in order to describe but not intended to limit, concrete details such as specific system structure, technique, and so on are proposed, thereby facilitating comprehensive understanding of the embodiments of the present invention. In some other conditions, detailed explanations of method, circuit, device and system well known to the public are omitted, so that unnecessary details can be prevented from obstructing the description of the present invention.

It should be understood that, when a term "comprise" is used in the description and annexed claims, the term "include" indicates existence of the described characteristics, the whole, steps, operations, elements and/or components, but not exclude existence or adding of one or a plurality of other characteristics, the whole, steps, operations, elements, components and/or combination thereof.

In order to describe the technical solutions of present invention, the technical solutions of the present invention are described below with reference to specific embodiments.

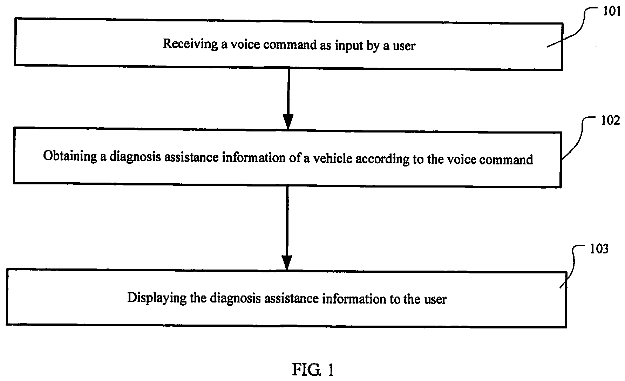

Example 1 of the present invention provides an intelligent diagnosis assistance method. FIG. 1 illustrates a flow chart of an intelligent diagnosis assistance method provided by this example of the present solution. As shown in FIG. 1, the method in this example can include following steps:
step 101, receiving a voice command as input by a user.

An executive subject in the method of the present invention can be an intelligent diagnosis assistance equipment, this equipment can be provided with a voice-input device such as a microphone, etc. Particularly, a user can input a voice command by using the voice-input device. The equipment can be a mobile phone, a tablet, a computer, etc., and can also be a vehicle diagnostic apparatus.

Step 102, acquiring the diagnosis assistance information of the vehicle according to the voice command.

Wherein, the vehicle can be the one that is ready to be detected, can be the one that is being detected, and can also be one which the user is interested in, it is not limited in this example.

The diagnosis assistance information can be any information that is used for assisting the user in performing diagnosis and maintenance; for example, the diagnosis assistance information can include any one of following information: archival information, circuit diagram, accessary information, maintenance method information, maintenance record, information of maintenance experience, fault code analysis information, etc. Wherein, the archival information can include any one of the following information: a vehicle type, a year model, a manufacturer, an engine type, a vehicle owner information, etc.

The diagnosis assistance information can be stored in the device, and can be stored in the server, the device can obtain the diagnosis assistance information by communicating with the server.

Concrete content of the voice command can be set according to an actual need. For example, the voice command can be an instruction used for instructing a starting of searching for the diagnosis assistance information, such as "start to search", etc., the user can input the instruction by way of voice, after receiving the instruction, the device can search the diagnosis assistance information of the vehicle. For another example, the voice command can be "start to diagnose", when receiving the instruction, the device starts to perform a diagnosis for the vehicle and obtain the diagnosis assistance information simultaneously.

Step 103, displaying the diagnosis assistance information to the user.

After the diagnosis assistance information is obtained, the diagnosis assistance information can be displayed to the user. There are various presenting methods, it is not limited herein in this example.

Optionally, said displaying the diagnosis assistance information to the user can include any one of following operations: displaying the diagnosis assistance information to the user; broadcasting the diagnosis assistance information to the user; transmitting the diagnosis assistance information to a user terminal, such that the user terminal can broadcast and/or present the diagnosis assistance information to the user.

Correspondingly, the device can be provided with at least one of following devices: a voice broadcasting device such as a loudspeaker, etc.; a display device such as a display screen, etc.; a communication device such as a Wi-Fi device, a blue tooth device, etc. The device can be the vehicle diagnostic apparatus, the user terminal can be a mobile phone of the user, after the device obtains the diagnosis assistance information, it can transmit the diagnosis assistance information to the cellphone, such that the mobile phone can broadcast and/or present the diagnosis assistance information to the user.

Preferably, said displaying the diagnosis assistance information to the user can particularly comprise:
broadcasting the diagnosis assistance information to the user in a form of voice, and displaying the diagnosis assistance information on an interface in the form of graphics or text.

For example, the diagnosis assistance information can include a circuit diagram, etc., the device can broadcast information such as a principle of the circuit diagram, parameters of elements, connection ways, etc. to the user, and can display the circuit diagram on the interface, thereby providing convenience for the user to look over textual and graphic files, and improving an efficiency and an accuracy of maintenance.

Wherein, the interface can be a user interface of an executive subject (i.e., the device) of the method in this example. The user in this example can be referred to as the vehicle maintenance personnel, and can also be other users such as a vehicle owner, etc.

In the intelligent diagnosis assistance method provided by this example, by receiving the voice command as input by the user, acquiring the diagnosis assistance information of the vehicle according to the voice command, and displaying the diagnosis assistance information to the user, such that the user can search the diagnosis assistance information of the vehicle by way of voice before or when performing the maintenance, there is no need to look over a maintenance manual manually, the efficiency of maintenance is improved, user's time is saved, and conveniences are brought to the user.

### Embodiment I

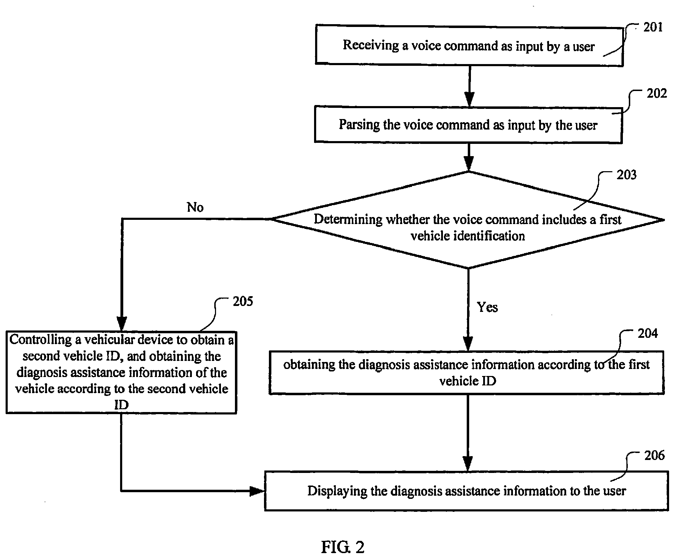

Embodiment I of the present invention provides an intelligent diagnosis assistance method. FIG. 2 illustrates a flow chart of an intelligent diagnosis assistance method provided by embodiment I of the present solution. As shown in FIG. 2, the method in this embodiment can comprise:
Step 201, receiving a voice command as input by a user.

Step 202, parsing the voice command.

Step 203, judging whether the voice command include a first vehicle ID (Identification). If the voice command includes the first vehicle identification, executing step 204, if the voice command doesn't include the first vehicle ID, executing step 205.

Particularly, after the voice command as input by the user is obtained, the voice command can be parsed, and whether the voice command includes the first vehicle identification is judged.

The first vehicle ID can include at least one of the following information: a vehicle license plate number, a VIN (Vehicle Identification Number), a system ID in the vehicle.

The VIN is consisted of a plurality of symbols, and includes information such as a manufacturer of the vehicle, years, a vehicle model, vehicle body type and code, motor code and assembly site, etc., and is of great importance for accurate diagnosis and maintenance of the vehicle. The system ID can be a name, a type, or a code of the system. The system can be any system or assembly in the vehicle such as a motor system, a ABS (Antilock Braking System), and so on.

For example, the user can input "vehicle license plate number, Jing AXXXXX" by way of voice, the device can, according to the voice command as input by the user, parse the first vehicle ID "Jing AXXXXX" out of the voice command.

Step 204, acquiring the diagnosis assistance information of the vehicle according to the first vehicle identification, and then executing step 206.

Said acquiring the diagnosis assistance information of the vehicle can comprise:
Submitting a diagnosis assistance information acquisition request to a server, wherein a diagnosis assistance information database is preset in the server; receiving the diagnosis assistance information returned by the server.

Particularly, the diagnosis assistance information acquisition request can include the first vehicle ID, the diagnosis assistance information database is preset in the server, the server can search the corresponding diagnosis assistance information in the diagnosis assistance information database according to the first vehicle ID.

Optionally, the diagnosis assistance information database can include a plurality of databases that is used for storing many diagnosis assistance information. For example, the plurality of databases can include a vehicle archive database configured to store archive information, a historical diagnosis database configured to store historical diagnostic information, and a maintenance database configured to store maintenance information respectively.

Since the diagnosis assistance information needs to be accumulated over a long period, thus, it is possible to cooperate with the existing maintenance information companies and data companies to integrate all diagnosis assistance information. Further, it is also possible to establish a retrieval data correspondence database configured to retrieve all these databases described above; meanwhile, an organization and a retrieval of each of the databases are designed according to a four-layered sequence, that is, a manufacturer, a vehicle type, a year model, and an engine type.

Particularly, when the server obtains the first vehicle ID, it can search the manufacturer, the vehicle model, the year model, and the engine type of the vehicle in the retrieval data correspondence database according to the first vehicle identification, and then search the various databases according to the manufacturer, the vehicle model, the year model, and the engine type of the vehicle, thereby acquiring the detailed diagnosis assistance information.

By setting the plurality of databases at a server side, a search can be performed more conveniently, and a search result can be more accurate and more reliable.

Furthermore, the device can also upload information of the vehicle to the server for the server to update database. The information of the vehicle includes but is not limited to: a diagnostic result of the vehicle, record data of the vehicle such as freeze frame data, geographic location data, etc. A self-learning functionality of the database can be implemented by uploading data, such that the diagnosis assistance information in the database can be complemented and corrected continually.

Step 205, acquiring a second vehicle ID (Identification) according to the vehicle-mounted device, and acquiring the diagnosis assistance information of the vehicle according to the second vehicle ID, and then executing step 206.

If the voice command doesn't include the first vehicle ID, the second vehicle ID can be obtained automatically.

Particularly, the intelligent diagnosis assistance device can be provided therein with an OBD (On-Board Diagnosis) interface, or as an alternative, this device can be connected to the OBD interface. The OBD interface is an on-line diagnostic interface of the vehicle, and can be used for implementing functionalities such as reading vehicle data, writing procedures, etc.

The vehicle-mounted device can be the device in the vehicle, which is configured to be connected with the intelligent diagnosis assistance device, the intelligent diagnosis assistance device can be connected with the vehicle-mounted device through the OBD interface, and obtain the second vehicle ID through the vehicle-mounted device. The second vehicle ID can be such as the vehicle license plate number or the VIN, etc.

Of course, except for the method of OBD, the vehicle-mounted device can also communicate with the intelligent diagnosis assistance device by other ways, which is not limited in this embodiment. For example, the vehicle-mounted device can pre-store the second vehicle ID, and the vehicle-mounted device can transmit the second vehicle ID to the intelligent diagnosis assistance device by means of, such as Wi-Fi.

After the intelligent diagnosis assistance device obtains the second vehicle ID, it can obtain the diagnosis assistance information of the vehicle according to the second vehicle ID. In this step which is similar to step 203, the diagnosis assistance information acquisition request can be submitted to the server, and the diagnosis assistance information returned by the server can be received. Wherein, the diagnosis assistance information acquisition request can include the second vehicle identification, such that the server can return back the corresponding diagnosis assistance information according to the second vehicle ID.

In this embodiment, said acquiring the diagnosis assistance information of the vehicle according to the voice command can be implemented by executing steps 202-205.

Step 206, displaying the diagnosis assistance information to the user.

A concrete implementation principle of step 207 in this embodiment is similar to that of step 103 in example I, it is not repeatedly descried herein.

In the intelligent diagnosis assistance method provided in this embodiment, by parsing the voice command, judging whether there exists the first vehicle ID in the voice command, acquiring the diagnosis assistance information of the vehicle according to the first vehicle ID if there exists the first vehicle ID in the voice command, such that the user can input information that he/she wants to search by way of voice, a customized need of the user can be satisfied; if there doesn't exist the first vehicle ID, by controlling the vehicle-mounted device to obtain the second vehicle ID, and acquiring the diagnosis assistance information of the vehicle according to the second vehicle ID, such that the diagnosis assistance information which is corresponding to the vehicle and is ready to be diagnosed currently or is being diagnosed can be obtained fast and accurately, it is unnecessary for the user to memorize an identifier of the vehicle, there is a stronger applicability.

### Embodiment II

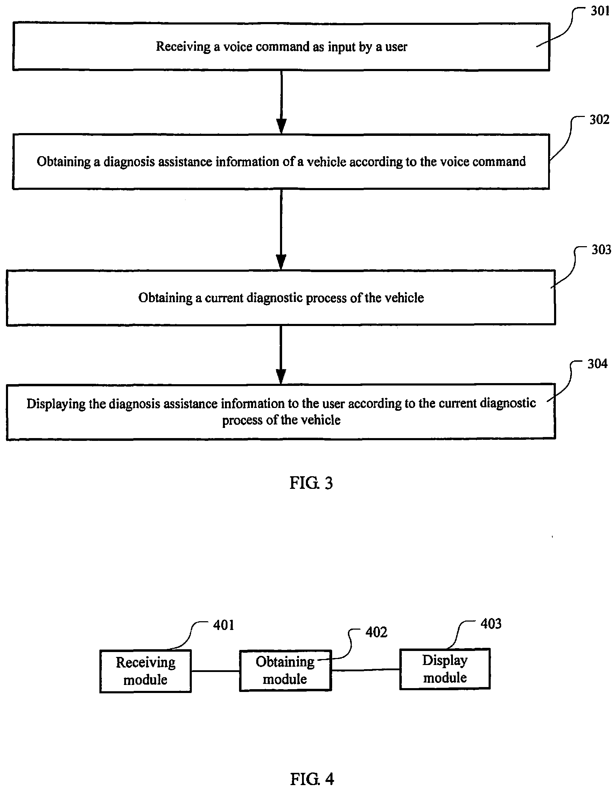

Embodiment II of the present solution provides an intelligent diagnosis assistance method. FIG. 3 illustrates a flow chart of the intelligent diagnosis assistance method provided by Embodiment II of the present solution. As shown in FIG. 3, the method in this embodiment can include:
Step 301, receiving a voice command as input by a user.

Step 302, acquiring diagnosis assistance information according to the voice command.

In this embodiment, regarding concrete implementation principle of steps 301-302, reference can be made to the aforesaid various embodiments, it is not repeatedly described herein.

Step 303, acquiring current vehicle diagnosis process.

Step 304, displaying diagnosis assistance information to the user according to the current vehicle diagnosis process.

In this embodiment, the intelligent diagnosis assistance device can present the diagnosis assistance information to the user according to a diagnosing process. Particularly, the intelligent diagnosis assistance device can be connected to the vehicle through the OBD interface, and perform a diagnosis for the vehicle.

Optionally, the diagnosing process can include phases such as initialization, processing, and ending, etc. Methods of presenting the diagnosis assistance information corresponding to different phases may be different.

In an initialization phase, the voice command can be parsed, fault description information in the voice command is obtained, and information of a system or an assembly which may malfunctions is broadcasted to the user according to the fault description information, and information including a circuit diagram and relevant maintenance information of the system or the assembly is displayed.

For example, the user can input "the front of the vehicle leaks water" by way of voice, this device judges that it is an engine system which may malfunction according to the fault description, and can broadcast this judgment to the user by way of voice, and display textual and graphical information of the engine system simultaneously.

In the processing phase, fault codes, diagnosis fault information can be broadcasted, and a dynamic circuit diagram, fault information and etc. are displayed in a graphical and a textual form.

Optionally, in a diagnosing process, the diagnosis assistance information of the system which is being diagnosed currently in the vehicle can be obtained, and the diagnosis assistance information of the system is displayed to the user.

For example, if the system that is being diagnosed currently is the engine system, it is possible to transmit an identification information of the engine system to the server, thereby acquiring the diagnosis assistance information of the engine system including the circuit diagram of the engine system, the maintenance files, and presenting the diagnosis assistance information of the engine system to the user, and thereby providing convenience for the user to perform a diagnosis and a maintenance for the vehicle.

In an ending phase, information including a diagnostic report and a clear code result can be broadcasted and displayed on an interface in a textual and graphic way.

Optionally, information of accessories of the vehicle that need to be changed can also be determined according to the diagnostic result of the vehicle.

Particularly, by performing the diagnosis for the vehicle, which accessories of the vehicle malfunction can be determined, these blooey accessories can be considered as the ones that need to be switched. The accessory information can be a name of the accessory, a model of the accessory, etc. After the information of the accessories that need to be switched is obtained, the information of the accessories can be transmitted to the server by which commodity information corresponding to the accessory information is searched in an on-line shopping mall.

The accessory information can correspond to one or a plurality of commodity information. Wherein, the commodity information corresponding to the accessory information can be the one which contains the accessory information and is provided by a merchant; for example, if the accessory information is a "temperature sensor", the commodity information can be the one that contains the word of "temperature sensor". Alternatively, the commodity information corresponding to the accessory information can be the one which has a similarity to the accessory information larger than a preset threshold value. The commodity information can include the name of the commodity, the model of the commodity, etc., and can include a price of the commodity, a place of dispatch, a direction for use, etc.

After the commodity information is obtained, the commodity information can be displayed to the user. For example, information such as "the blooey accessory is the temperature sensor, N types of temperature sensors are searched in the on-line shopping mall, they are 1: XX brand temperature sensor which has a price of XX Yuan and is delivered from Beijing, 2: XX brand temperature sensor which has a price of XX Yuan and is delivered from Shanghai;...... etc. respectively". Or as an alternative, the commodity information and the corresponding purchase link can be displayed to the user directly, so that the user can acquire the detailed information of the accessories.

Furthermore, the commodity information which is selected from the commodity information and is expected to be purchased by the user can also be received; a delivery information is obtained according to the commodity information as input by and expected to be purchased by the user, and an order information is generated according to the commodity information expected to be purchased and the delivery information; the order information can be transmitted to the server, such that the server can allocate delivery tasks according to the order information.

According to said assumption, after the commodity information is broadcasted to the user, the user can input "selecting a first temperature sensor" or "selecting XX brand temperature sensor" by way of voice, then, the device can transmit the order information to the server according to the commodity information selected by the user and the delivery information, thereby implementing a selective purchasing of the accessories.

The delivery information can include a delivery position information and/or a contact way of the user. After the server sends the delivery tasks, a delivery man or a delivery organization can deliver the commodity selected to be purchased by the user to a corresponding location, or contact and communicate with the user regarding matters on delivery.

Optionally, said acquiring the delivery information, and generating the order information according to the information of commodity that is expected to be purchased, and the delivery information can include: acquiring geographic location information of the vehicle, and generating the order information according to the geographic location information and the information of the commodity that is expected to be purchased.

In this way, the delivery man or the delivery organization can deliver the commodity that is selected to be purchased by the user to the place where the vehicle is located, it is convenient for the user to replace accessories for the vehicle directly, and an efficiency of replacement of accessories is improved.

As an alternative, said acquiring the delivery information, and generating the order information according to the information of commodity that is expected to be purchased and the delivery information can comprise: transmitting a search instruction to the server, wherein the search instruction includes a vehicle ID, such that the serve can search the vehicle owner information of the vehicle according to the search instruction; receiving the vehicle owner information sent by the server, and generating the order information according to the vehicle owner information and the information of commodity expected to be purchased.

The vehicle owner information can include a name, a telephone number, an address of the vehicle owner, etc. The vehicle identification information can be obtained according to an OBD (On-Board Diagnosis) interface or by other ways.

In this way, the delivery man or the delivery organization can communicate with the vehicle owner regarding delivery matters directly, there is no need for the user to input information including the contact way, convenience is brought to the user.

In the intelligent diagnosis assistance method provided by this embodiment, by acquiring the current vehicle diagnosing process, and displaying the diagnosis assistance information to the user according to the current vehicle diagnosing process, such that presenting of the diagnosis assistance information is adaptive to the diagnosing process, and the efficiency of diagnosis and maintenance is improved.

Based on the technical solutions provided by the various embodiments described above, preferably, when the diagnosis assistance information includes at least two types, a pushing of the diagnosis assistance information can be implemented by executing a plurality of steps.

Particularly, the voice command as input by the user can be obtained firstly, and the diagnosis assistance information of the vehicle is obtained according to the voice command, when the diagnosis assistance information includes at least two types, the type of the diagnosis assistance information can be broadcasted to the user firstly, such that the user can choose detailed information he/she wants to look over according to the type of the diagnosis assistance information, conveniences are brought to the user.

Optionally, this type can be a classification of the diagnosis assistance information, can include, for example, a circuit diagram, an accessory information, a maintenance method, a maintenance record, a maintenance experience information, a fault code parsing information, and so on.

Correspondingly, after acquiring the diagnosis assistance information, this device can broadcast "please choose a classification that is expected to be watched and listed as follows: the circuit diagram, the accessory information, the maintenance method, the maintenance record, the maintenance experience information, the fault code parsing information", and then receive the voice command as input by the user again, determine the classification which is expected to be watched and chosen by the user from the types of the diagnosis assistance information according to the voice command received once again. For example, when the user wants to look over a maintenance record of the vehicle, he/she can input "maintenance record" by way of voice, this device can broadcast a corresponding maintenance record to the user according to the voice command as input by the user, the broadcasting can either be a voice broadcast or be a video broadcast. When the type selected by the user is a circuit diagram, a corresponding dynamic circuit diagram can be broadcasted.

Optionally, said type can also be system information corresponding to the diagnosis assistance information, for example, said type can be "of the engine system, of the ABS (Anti-block braking System), of an electrical control system", etc.

Correspondingly, the device can broadcast "please choose an option that is expected to be watched: a diagnosis assistance information of the engine system, a diagnosis assistance information of the ABS, a diagnosis assistance information of the electrical control system" to the server by way of voice; the user can input the option that he/she wants to watch by way of voice, such as "engine system" or "the diagnosis assistance information of the engine system", the device can broadcast the diagnosis assistance information of the engine system to the user according to the voice command as input by the user.

Based on the technical solutions provided by the various embodiments described above, preferably, it is also possible to receive diagnostic procedure information as input by the user, and perform the corresponding diagnostic procedure according to the diagnostic procedure information. The diagnostic procedure can include one of the following operations: reading fault codes, clearing fault codes, reading system information, movement testing, ending diagnosis, watching report, printing report, etc.

For example, the user can input "reading fault code" by way of voice, the device can then perform a fault code reading operation. In this way, each step in the diagnosing process can be controlled by the user by way of voice, conveniences are brought to the user, and an operability of diagnosis is improved.

Based on the technical solutions provided by the various embodiments, preferably, it is also possible to obtain a diagnostic result of the vehicle and transmit the diagnostic result to the server, such that the server can search the corresponding maintenance help information according to the diagnostic result; the maintenance help information sent by the server can be received, and the maintenance help information can be pushed to the user.

On a server side, the diagnostic result and the maintenance help information can be stored in association. After the diagnostic result of the vehicle that is to be diagnosed is obtained, the corresponding maintenance help information can be obtained from the server, which helps the user to carry out maintenance aiming at the fault that has been diagnosed, an efficiency and an accuracy of maintenance are improved.

Based on the technical solutions provided by the various embodiments, preferably, a diagnostic process of the vehicle can be an intelligent and automatic diagnostic process. The intelligent and automatic process can include: acquiring the vehicle ID, such as the vehicle plate information or the vehicle identification code, and transmitting the vehicle ID to the server; the server can search the vehicle type information corresponding to the vehicle ID in a database, and control the device to initiate a corresponding diagnostic software, thereby performing a diagnosis for the vehicle.

Different diagnostic software is applicable to different types of vehicles; for example, vehicle type A corresponds to a diagnostic software 1, vehicle type B corresponds to a diagnostic software 2, if it is detected that the vehicle type information that corresponds to the current vehicle is A, the diagnostic software 1 is launched so as to perform the diagnosis for the vehicle. In the intelligent self-diagnosis process, by acquiring the vehicle ID information, the corresponding diagnostic software can be initiated automatically, there is no need for the user to input vehicle information manually.

In the technical solutions provided by the various embodiments described above, parsing the voice command as input by the user can be accomplished by voice recognition software which can convert the voice command as input by the user into a corresponding text or command. In an actual application, the voice recognition software can resident in the background, when the user needs to search the diagnosis assistance information, he/she only needs to speak a voice command such as "maintenance help", then, steps in any one of the embodiments described above can be started to obtain the diagnosis assistance information and broadcast the diagnosis assistance information to the user in way of voice. At the time of voice broadcast, the user can also look over textual and graphical files, an interruption of a diagnosis of the vehicle is unnecessary.

It should be understood that, value of serial number of the steps in the aforesaid embodiment doesn't mean a sequencing of execution sequences of the steps, the execution sequence of each of the steps should be determined by functionalities and internal logics of the steps themselves, and shouldn't be regarded as limitation to an implementation process of the embodiment of the present invention.

### Embodiment III

Embodiment III of the present solution provides an intelligent diagnosis assistance device. FIG. 4 illustrates a schematic block diagram of the intelligent diagnosis assistance device provided by Embodiment III of the present solution. For describing conveniently, the part that is related with the embodiment of the present solution is illustrated merely. The intelligent diagnosis assistance device can be a software unit, a hardware unit, or a unit of combination of software and hardware arranged in the intelligent diagnosis assistance device, it can also be taken as an independent pendant which is integrated into the intelligent diagnosis assistance device.

As shown in FIG. 4, the device in this embodiment can comprise:
a receiving module 401 configured to receive a voice command as input by a user;
an obtaining module 402 configured to obtain a diagnosis assistance information of a vehicle according to the voice command; and
a display module 403 configured to present the diagnosis assistance information to the user.

The intelligent diagnosis assistance device in this embodiment can be used for performing the intelligent diagnosis assistance method in any one the embodiments described above, regarding a detailed implementation principle, please refer to any one of the aforesaid embodiments, it is not repeatedly described herein.

By receiving, via the intelligent diagnosis assistance device provided by this embodiment, the voice command as input by the user, acquiring the diagnosis assistance information according to the voice command, and displaying the diagnosis assistance information to the user, such that the user can search the diagnosis assistance information by way of voice before or when performing a maintenance, there is no need to look over a maintenance manual manually, an efficiency of maintenance is improved, user's time is saved, and conveniences are brought to the user.

The obtaining module 402 is particularly configured to:
parse the voice command;
determine whether the voice command includes a first vehicle identification; and
obtain the diagnosis assistance information according to the first vehicle identification if the voice command includes the first vehicle identification; or
control a vehicle-mounted device to obtain a second vehicle identification, and obtain the diagnosis assistance information of the vehicle according to the second vehicle identification if the voice command doesn't include the first vehicle identification.

Wherein, said acquiring the diagnosis assistance information of the vehicle comprises:
submitting a diagnosis assistance information acquisition request to a server; wherein a diagnosis assistance information database is preset in the server; and
receiving the diagnosis assistance information returned by the server.

Optionally, the display module 403 can be particularly configured to:
broadcast the diagnosis assistance information to the user in a form of speech, and display the diagnosis assistance information on an interface in the form of graphics or text.

Optionally, the display module 403 can be particularly configured to:
obtain a current vehicle diagnostic process; and
present the diagnosis assistance information to the user according to the current vehicle diagnostic process.

Optionally, the display module 403 can be also configured to:
determine information of an accessory that needs to be replaced according to a diagnostic result of the vehicle;
obtain a commodity information corresponding to the information of the accessory in an on-line shopping mall; and
display or broadcast the commodity information to the user.

Optionally, the display module 403 can also be configured to:
receive an information of a commodity which is selected from the commodity information and is expected to be purchased by the user;
obtain a delivery information according to the information of the commodity as input by and expected to be purchased by the user, and generate an order information according to the information of the commodity expected to be purchased and the delivery information; and
transmit the order information to the server, such that the server can assign delivery tasks according to the order information.

It can be clearly understood by one or ordinary skill in the art that, for describing conveniently and concisely, dividing of the aforesaid various functional units, functional modules is exemplified merely, in an actual application, the aforesaid functions can be assigned to different functional units and functional modules to be accomplished, that is, an inner structure of a data synchronizing device is divided into functional units or modules so as to accomplish the whole or a part of functionalities described above. The various functional units, modules in the embodiments can be integrated into a processing unit, or each of the units exists independently and physically, or two or more than two of the units are integrated into a single unit. The aforesaid integrated unit can by either realized in the form of hardware or in the form of software functional units. In addition, specific names of the various functional units and modules are only used for distinguishing from each other conveniently, but not intended to limit the protection scope of the present invention. Regarding a specific working process of the units and modules in the aforesaid device, please refer to a corresponding process in the aforesaid method embodiments, it is not repeatedly described herein.

### Embodiment IV

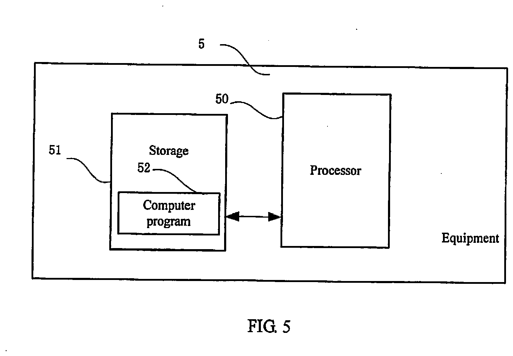

Embodiment IV of the present solution provides an intelligent diagnosis assistance equipment. FIG. 5 illustrates a schematic block diagram of the intelligent diagnosis assistance equipment provided by embodiment IV of the present solution. As shown in FIG. 5, the equipment 5 in this embodiment comprises: one or a plurality of processor(s) 50, a storage device 51 and computer program 52 stored in the storage device 51 and can be executed by the processor 50. The processor 50 implements steps in the various intelligent diagnosis assistance method embodiments when executing the computer program 52.

As an alternative, when the processor 50 executes the computer program 50, functionalities of the various modules/units in the aforesaid device embodiments such as the functionalities of modules 401-404 shown in FIG. 4 are achieved.

The equipment 5 can include but is not limited to: the processor 50, and the storage device 51. It can be understood for one of ordinary skill in the art that, FIG. 5 is merely an example of the equipment 5, and is not constituted as limitation to the equipment 5, more or less components shown in FIG. 5 can be included, or some components or different components can be combined; for example, the terminal device for determining wellbore cross-sectional shape can also include an input and output device, a network access device, a bus, etc.

The so called processor 50 can be CPU (Central Processing Unit), and can also be other general purpose processor, DSP (Digital Signal Processor), ASIC (Application Specific Integrated Circuit), FGPA (Field-Programmable Gate Array), or some other programmable logic devices, discrete gate or transistor logic device, discrete hardware component, etc. The general purpose processor can be a microprocessor, or alternatively, the processor can also be any conventional processor and so on.

The storage device 51 can be an internal storage unit of the equipment 5, such as a hard disk or a memory of the equipment 5. The storage device 51 can also be an external storage device of the equipment 5, such as a plug-in hard disk, a SMC (Smart Media Card), a SD (Secure Digital) card, a FC (Flash Card) equipped on the equipments. Further, the storage device 51 may include both the internal storage unit and the external storage device of the equipment 5, either. The storage device 51 is configured to store the computer programs, and other procedures and data needed by the equipment 5. The storage device 51 can also be configured to store data that has been output or being ready to be output temporarily.

Preferably, the equipment 5 can be cellphone, a tablet device, a computer, etc., and can be a diagnostic equipment such as the diagnostic equipment with the type of X431 PRO/PRO3S/PADIII and manufactured by SHENZHEN LAUNCH SOFTWARE CO., LTD.

Optionally, the equipment 5 can also include a diagnostic module configured to be connected to the vehicle, an interface of the diagnostic module can be OBDII-16 (i.e., a diagnostic interface having 16 standard pins and opened by OBD), the diagnostic module can include a physical layer and a link layer communicated with vehicle communication protocol, the link layer includes all vehicle diagnosis communication protocols.

Optionally, the equipment 5 can also include a network communication device which can be a 3G module, a 4G module, a 5G module, a Wi-Fi module, etc., which is configured to implement a communication with the server.

Optionally, the equipment 5 can also comprise a positioning device, the positioning device can include at least one of the following modules: GPS (Global Positioning System) module, AGPS (Assisted Global Positioning System), BD (Beidou Navigation Positioning System) module, GLONASS satellite navigation module, etc. which are configured to obtain geographical positioning information.

Optionally, the equipment 5 can also include a voice inputting device which can be such as a microphone that supports the voice command as input by the user, and so on.

Optionally, the equipment 5 can also comprise a broadcasting device, the broadcasting device can be a loudspeaker, etc. which is configured to broadcast the diagnosis assistance information.

Optionally, the equipment 5 can also comprise a displaying device, the displaying device can be a display screen, etc. which is configured to display the diagnosis assistance information.

An embodiment of the present solution further provides a computer readable storage medium which stores computer program, when the computer program is executed by the processor, steps in the method of intelligent diagnosis assistance method in any one of the aforesaid embodiments are implemented.

In the embodiments of the present invention, the descriptions of the embodiments in the present invention are emphasized respectively, in regard to the part without detailed description in some embodiments, please refer to related description in other embodiments.

One of ordinary skill in the art will notice that, the elements and algorithm steps of each of the examples described in connection with the embodiments disclosed herein can be implemented in electronic hardware, or in combination with computer software and electronic hardware. Whether these functions are implemented by hardware or software depends on the specific application and design constraints of the technical solution. The skilled people could use different methods to implement the described functions for each particular application, but such implementations should not be considered as going beyond the scope of the present invention, as solely defined by the appended claims.

It should be understood that, in the embodiments of the present invention, the disclosed device/terminal device and method could be implemented in other ways. For example, the device described above are merely illustrative; for example, the division of the units is only a logical function division, and other division could be used in the actual implementation, for example, multiple units or components could be combined or integrated into another system, or some features can be ignored, or not performed. In another aspect, the coupling or direct coupling or communicating connection shown or discussed could be an indirect, or a communicating connection through some interfaces, devices or units, which could be electrical, mechanical, or otherwise.

The units described as separate components could or could not be physically separate, the components shown as units could or could not be physical units, which can be located in one place, or can be distributed to multiple network elements. Parts or all of the elements could be selected according to the actual needs to achieve the object of the present embodiment.

In addition, the various functional units in each of the embodiments of the present invention can be integrated into a single processing unit, or exist individually and physically, or two or more than two units are integrated into a single unit. The aforesaid integrated unit can either be achieved by hardware, or be achieved in the form of software functional units.

If the integrated unit is achieved in the form of software functional units, and is sold or used as an independent product, it can be stored in a computer readable storage medium. Based on this understanding, a whole or part of flow process of implementing the method in the aforesaid embodiments of the present invention can also be accomplished by the computer programs configured to instruct relevant hardware. When the computer program is executed by the processor, the steps in the various method embodiments described above can be implemented. Wherein, the computer program comprises computer program codes, which can be in the form of source code, object code, executable documents or some intermediate form, etc. The computer readable medium can include: any entity or device that can carry the computer program codes, recording medium, USB flash disk, mobile hard disk, hard disk, optical disk, computer storage device, ROM (Read-Only Memory), RAM (Random Access Memory), electrical carrier signal, telecommunication signal and software distribution medium, etc. It needs to be explained that, the contents contained in the computer readable medium can be added or reduced appropriately according to the requirement of legislation and patent practice in a judicial district, for example, in some judicial districts, according to legislation and patent practice, the computer readable medium doesn't include electrical carrier signal and telecommunication signal.

## Claims

1. An intelligent diagnosis assistance method, comprising:
receiving a voice command input by a user (201); and
parsing the voice command (202);
**characterized in that**, the method further comprises:
determining whether a first vehicle identification is included in the voice command (203); and
if it is determined that the first vehicle identification is included in the voice command, the method further comprises:
submitting a diagnosis assistance information acquisition request including the first vehicle identification to a server; wherein a diagnosis assistance information database is present in the server; and acquiring the diagnosis assistance information (204) corresponding to the first vehicle identification from the server by searching in the diagnosis assistance information database; or
if it is determined that the first vehicle identification is not included in the voice command, the method further comprises:
controlling a vehicle-mounted device to acquire a second vehicle identification, and acquiring (205) the diagnosis assistance information of the vehicle from the server according to the second vehicle identification;
wherein the method further comprises: displaying the diagnosis assistance information to the user (206);
wherein diagnostic results and maintenance help information are stored in association in the server, the method further comprises:
acquiring a diagnostic result of the vehicle and transmitting the diagnostic result to the server in order that the server searches maintenance help information corresponding to the diagnostic result of the vehicle; and
receiving the maintenance help information sent by the server and pushing the maintenance help information to the user.

2. The method according to claim 1, **characterized in that**, said displaying the diagnosis assistance information to the user particularly comprises:
sending the diagnosis assistance information to the user in a form of voice, and displaying the diagnosis assistance information on an interface in the form of graphics or text.

3. The method according to claim 2, **characterized in that**, said displaying the diagnosis assistance information to the user particularly comprises:
obtaining a current vehicle diagnostic process; and
displaying the diagnosis assistance information to the user according to the current vehicle diagnostic process.

4. An intelligent diagnosis assistance device, the intelligent diagnosis assistance device comprising:
a receiving module (401) configured to receive a voice command as input by a user; an obtaining module (402) configured to parse the voice command;
a display module (403) configured to present the diagnosis assistance information to the user;
the intelligent diagnosis assistance device **characterised in that**
the obtaining module (402) is further adapted to determine whether the voice command includes a first vehicle identification and, if it is determined that the first vehicle identification is included in the voice command, the obtaining module (402) is adapted to submit a diagnosis assistance information acquisition request including the first vehicle identification to a server and to obtain diagnosis assistance information of a vehicle corresponding to the first vehicle identification from the server; wherein a diagnosis assistance information database is present in the server; or
if it is determined that the first vehicle identification is not included in the voice command, the obtaining module (402) is adapted to control a vehicle-mounted device so as to acquire a second vehicle identification and to obtain the diagnosis assistance information of the vehicle according to the second vehicle identification from the server;
wherein diagnostic results and maintenance help information are stored in association in the server,
the obtaining module (402) is further configured to:
acquire a diagnostic result of the vehicle and transmit the diagnostic result to the server in order for the the server to search maintenance help information corresponding to the diagnostic result of the vehicle;
receive the maintenance help information sent by the server and push the maintenance help information to the user.

5. The device according to claim 4, **characterized in that**, the display module (403) is particularly configured to:
send the diagnosis assistance information to the user in a form of voice, and display the diagnosis assistance information on an interface in the form of graphics or text.

6. The device according to claim 4, **characterized in that**, the display module (403) is particularly configured to:
obtain a current vehicle diagnostic process; and
present the diagnosis assistance information to the user according to the current vehicle diagnostic process.

7. An intelligent diagnosis assistance equipment (5) comprising the intelligent diagnosis assistance device of claim 4 and means adapted to execute the steps of method claims 1-3.

8. A computer readable storage medium which stores a computer program (52) comprising instructions to cause the intelligent diagnosis assistance equipment of claim 7 to execute the steps of method claims 1-3.

## Patentansprüche

1. Intelligentes diagnostisches Hilfsverfahren, das Folgendes umfasst:
Empfangen eines Sprachbefehls, eingegeben durch einen Benutzer (201); und
Parsen des Sprachbefehls (202);
**dadurch gekennzeichnet, dass** das Verfahren ferner Folgendes umfasst:
Bestimmen, ob eine erste Fahrzeugidentifikation im Sprachbefehl (203) enthalten ist; und wobei, wenn bestimmt wird, dass die erste Fahrzeugidentifikation im Sprachbefehl enthalten ist, das Verfahren ferner Folgendes umfasst:
Übermitteln einer diagnostischen Hilfsinformations-Erfassungsanforderung, umfassend die erste Fahrzeugidentifikation, an einen Server; wobei eine diagnostische Hilfsinformationsdatenbank im Server vorhanden ist; und Erfassen der diagnostischen Hilfsinformationen (204) entsprechend der ersten Fahrzeugidentifikation vom Server durch Suchen in der diagnostischen Hilfsinformationsdatenbank; oder
wobei, wenn bestimmt wird, dass die erste Fahrzeugidentifikation nicht im Sprachbefehl enthalten ist, das Verfahren ferner Folgendes umfasst:
Steuern einer am Fahrzeug montierten Vorrichtung zum Erfassen einer zweiten Fahrzeugidentifikation und Erfassen (205) der diagnostischen Hilfsinformationen des Fahrzeugs vom Server entsprechend der zweiten Fahrzeugidentifikation;
wobei das Verfahren ferner Folgendes umfasst: Anzeigen der diagnostischen Hilfsinformationen für den Benutzer (206) ;
wobei Diagnoseergebnisse und Wartungshilfsinformationen verknüpft im Server gespeichert werden, wobei das Verfahren ferner Folgendes umfasst:
Erfassen eines Diagnoseergebnisses des Fahrzeugs und Senden des Diagnoseergebnisses an den Server, damit der Server Wartungshilfsinformationen entsprechend dem Diagnoseergebnis des Fahrzeugs sucht; und
Empfangen der durch den Server gesendeten Wartungshilfsinformationen und Durchleiten der Wartungshilfsinformationen zum Benutzer.

2. Verfahren nach Anspruch 1, **dadurch gekennzeichnet, dass** das Anzeigen der diagnostischen Hilfsinformationen für den Benutzer insbesondere Folgendes umfasst:
Senden der diagnostischen Hilfsinformationen an den Benutzer in einer Form von Sprache und Anzeigen der diagnostischen Hilfsinformationen auf einer Schnittstelle in der Form von Grafik oder Text.

3. Verfahren nach Anspruch 2, **dadurch gekennzeichnet, dass** das Anzeigen der diagnostischen Hilfsinformationen für den Benutzer insbesondere Folgendes umfasst:
Erhalten eines aktuellen fahrzeugdiagnostischen Prozesses; und
Anzeigen der diagnostischen Hilfsinformationen für den Benutzer entsprechend dem aktuellen fahrzeugdiagnostischen Prozess.

4. Intelligente diagnostische Hilfsvorrichtung, wobei die intelligente diagnostische Hilfsvorrichtung Folgendes umfasst:
ein Empfangsmodul (401), ausgelegt zum Empfangen eines Sprachbefehls, wie durch einen Benutzer eingegeben;
ein Erhaltemodul (402), ausgelegt zum Parsen des Sprachbefehls;
ein Anzeigemodul (403) ausgelegt zum Präsentieren der diagnostischen Hilfsinformationen für den Benutzer;
wobei die intelligente diagnostische Hilfsvorrichtung **dadurch gekennzeichnet ist, dass** das Erhaltemodul (402) ferner angepasst ist zum Bestimmen, ob der Sprachbefehl eine erste Fahrzeugidentifikation umfasst und wobei, wenn bestimmt wird, dass die erste Fahrzeugidentifikation im Sprachbefehl enthalten ist, das Erhaltemodul (402) angepasst ist zum Übermitteln einer diagnostischen Hilfsinformations-Erfassungsanforderung, umfassend die erste Fahrzeugidentifikation, an einen Server und zum Erhalten von diagnostischen Hilfsinformationen eines Fahrzeugs entsprechend der ersten Fahrzeugidentifikation vom Server; wobei eine diagnostische Hilfsinformationsdatenbank im Server vorhanden ist; oder
wobei, wenn bestimmt wird, dass die erste Fahrzeugidentifikation nicht im Sprachbefehl enthalten ist, das Erhaltemodul (402) angepasst ist zum Steuern einer am Fahrzeug montierten Vorrichtung, um eine zweite Fahrzeugidentifikation zu erfassen und die diagnostische Hilfsinformation des Fahrzeugs entsprechend der zweiten Fahrzeugidentifikation vom Server zu erhalten;
wobei Diagnoseergebnisse und Wartungshilfsinformationen verknüpft im Server gespeichert werden, wobei das Erhaltemodul (402) ferner ausgelegt ist zum:
Erfassen eines Diagnoseergebnisses des Fahrzeugs und Senden des Diagnoseergebnisses an den Server, damit der Server Wartungshilfsinformationen entsprechend dem Diagnoseergebnis des Fahrzeugs sucht;
Empfangen der durch den Server gesendeten Wartungshilfsinformationen und Durchleiten der Wartungshilfsinformationen zum Benutzer.

5. Vorrichtung nach Anspruch 4, **dadurch gekennzeichnet, dass** das Anzeigemodul (403) insbesondere ausgelegt ist zum:
Senden der diagnostischen Hilfsinformationen an den Benutzer in einer Form von Sprache und Anzeigen der diagnostischen Hilfsinformationen auf einer Schnittstelle in der Form von Grafik oder Text.

6. Vorrichtung nach Anspruch 4, **dadurch gekennzeichnet, dass** das Anzeigemodul (403) insbesondere ausgelegt ist zum:
Erhalten eines aktuellen fahrzeugdiagnostischen Prozesses; und
Präsentieren der diagnostischen Hilfsinformationen für den Benutzer entsprechend dem aktuellen fahrzeugdiagnostischen Prozess.

7. Intelligente diagnostische Hilfsausrüstung (5), umfassend die intelligente diagnostische Hilfsvorrichtung nach Anspruch 4 und Mittel, die angepasst sind zum Ausführen der Schritte der Verfahrensansprüche 1-3.

8. Computerlesbares Speichermedium, das ein Computerprogramm (52) speichert, das Anweisungen umfasst, um die intelligente diagnostische Hilfsausrüstung nach Anspruch 7 zu veranlassen, die Schritte der Verfahrensansprüche 1-3 auszuführen.

## Revendications

1. Procédé d'aide au diagnostic intelligent, comportant les étapes consistant à :
recevoir une consigne vocale introduite par un utilisateur (201) ; et
analyser la consigne vocale (202) :
**caractérisé en ce que** le procédé comporte en outre les étapes consistant à :
déterminer si une première identification de véhicule est comprise dans la consigne vocale (203) ; et
s'il est déterminé que la première identification de véhicule est comprise dans la consigne vocale, le procédé comporte en outre les étapes consistant à :
soumettre une demande d'acquisition d'informations d'aide au diagnostic incluant la première identification de véhicule à un serveur ; une base de données d'informations d'aide au diagnostic étant présente dans le serveur ; et acquérir les informations d'aide au diagnostic (204) correspondant à la première identification de véhicule à partir du serveur en cherchant dans la base de données d'informations d'aide au diagnostic ; ou
s'il est déterminé que la première identification de véhicule n'est pas comprise dans la consigne vocale, le procédé comporte en outre les étapes consistant à :
commander un dispositif embarqué pour acquérir une seconde identification de véhicule, et acquérir (205) les informations d'aide au diagnostic du véhicule à partir du serveur d'après la seconde identification de véhicule ;
le procédé comportant en outre : le fait de présenter les informations d'aide au diagnostic à l'utilisateur (206) ;
des résultats de diagnostic et des informations d'aide à l'entretien étant stockés en association dans le serveur, le procédé comportant en outre les étapes consistant à :
acquérir un résultat de diagnostic du véhicule et transmettre le résultat de diagnostic au serveur afin que le serveur recherche des informations d'aide à l'entretien correspondant au résultat de diagnostic du véhicule ; et
recevoir les informations d'aide à l'entretien envoyées par le serveur et distribuer sélectivement les informations d'aide à l'entretien à l'utilisateur.

2. Procédé selon la revendication 1, **caractérisé en ce que** ladite présentation des informations d'aide au diagnostic à l'utilisateur comporte en particulier les étapes consistant à : envoyer les informations d'aide au diagnostic à l'utilisateur sous forme vocale, et afficher les informations d'aide au diagnostic sur une interface sous forme de graphique ou de texte.

3. Procédé selon la revendication 2, **caractérisé en ce que** ladite présentation des informations d'aide au diagnostic à l'utilisateur comporte en particulier les étapes consistant à :
obtenir un processus de diagnostic de véhicule actuel ;
et
présenter les informations d'aide au diagnostic à l'utilisateur selon le processus de diagnostic de véhicule actuel.

4. Dispositif d'aide au diagnostic intelligent, le dispositif d'aide au diagnostic intelligent comportant :
un module (401) de réception configuré pour recevoir une consigne vocale telle qu'introduite par un utilisateur ;
un module (402) d'obtention configuré pour analyser la consigne vocale ;
un module (403) d'affichage configuré pour présenter les informations d'aide au diagnostic à l'utilisateur ;
le dispositif d'aide au diagnostic intelligent étant **caractérisé en ce que**
le module (402) d'obtention est en outre prévu pour déterminer si la consigne vocale comprend une première identification de véhicule et, s'il est déterminé que la première identification de véhicule est comprise dans la consigne vocale, le module (402) d'obtention est prévu pour soumettre une demande d'acquisition d'informations d'aide au diagnostic incluant la première identification de véhicule à un serveur et pour obtenir des informations d'aide au diagnostic d'un véhicule correspondant à la première identification de véhicule à partir du serveur ; une base de données d'informations d'aide au diagnostic étant présente dans le serveur ; ou
s'il est déterminé que la première identification de véhicule n'est pas comprise dans la consigne vocale, le module (402) d'obtention est prévu pour commander un dispositif embarqué de façon à acquérir une seconde identification de véhicule et à obtenir les informations d'aide au diagnostic du véhicule d'après la seconde identification de véhicule à partir du serveur ;
des résultats de diagnostic et des informations d'aide à l'entretien étant stockés en association dans le serveur, le module (402) d'obtention étant en outre configuré pour :
acquérir un résultat de diagnostic du véhicule et transmettre le résultat de diagnostic au serveur afin que le serveur recherche des informations d'aide à l'entretien correspondant au résultat de diagnostic du véhicule ;
recevoir les informations d'aide à l'entretien envoyées par le serveur et distribuer sélectivement les informations d'aide à l'entretien à l'utilisateur.

5. Dispositif selon la revendication 4, **caractérisé en ce que** le module (403) d'affichage est configuré en particulier pour :
envoyer les informations d'aide au diagnostic à l'utilisateur sous forme vocale, et afficher les informations d'aide au diagnostic sur une interface sous forme de graphique ou de texte.

6. Dispositif selon la revendication 4, **caractérisé en ce que** le module (403) d'affichage est configuré en particulier pour :
obtenir un processus de diagnostic de véhicule actuel ;
et
présenter les informations d'aide au diagnostic à l'utilisateur selon le processus de diagnostic de véhicule actuel.

7. Équipement (5) d'aide au diagnostic intelligent comportant le dispositif d'aide au diagnostic intelligent selon la revendication 4 et des moyens prévus pour exécuter les étapes des revendications 1 à 3 de procédé.

8. Support de stockage lisible par ordinateur qui conserve un programme informatique (52) comportant des instructions destinées à amener l'équipement d'aide au diagnostic intelligent selon la revendication 7 à exécuter les étapes des revendications 1 à 3 de procédé.
